# EUROPÄISCHE PATENTSCHRIFT

(11) **EP 4 076 275 B1**
(45) Veröffentlichungstag und Bekanntmachung des Hinweises auf die Patenterteilung: **07.02.2024**
(21) Anmeldenummer: 20838929.6
(22) Anmeldetag: 15.12.2020
(51) Int. Cl.: A61F 2/07

(54) **MEHRFACHSTENT MIT MEMBRAN**
MULTIPLE STENT WITH MEMBRANE
ENDOPROTHÈSE MULTIPLE POURVUE D'UNE MEMBRANE

(30) Priorität: 20.12.2019 DE 102019135453
(43) Veröffentlichungstag der Anmeldung: 26.10.2022
(73) Patentinhaber: Stental GmbH, 79540 Lörrach (DE)
(72) Erfinder: NEUß, Malte, 53121 Bonn (DE)
(74) Vertreter: Schneiders & Behrendt Bochum
(86) Internationale Anmeldenummer: PCT/EP2020/086262
(87) Internationale Veröffentlichungsnummer: WO 2021/122626

(56) Entgegenhaltungen:
- EP-A2- 1 121 911
- EP-B1- 1 121 911
- WO-A1-96/33672
- DE-U1- 29 623 983
- US-A1- 2009 132 022

## Beschreibung

Die Erfindung betrifft einen Mehrfachstent mit mindestens zwei koaxial angeordneten Stents und mindestens einer Membran, wobei die Enden der Membran zwischen zwei Stents fixiert werden. Der Mehrfachstent dient insbesondere als Stentgraft zur Überbrückung von vaskulären Fehlbildungen, wie beispielsweise Aneurysmen oder Shunts, aber auch zur Verstärkung von labilen, brüchigen oder thrombotischen Gefäßwänden. Er wird ferner eingesetzt zum Wieder-Anschluss von aus gestenteten Gefäßen abzweigenden Gefäßen.

Stentgrafts zur Überbrückung von vaskulären Fehlbildungen sind in einer Vielzahl von Formen bekannt. In der Regel bestehen sie aus einem Stent, der mit einer Membran ganz oder teilweise überzogen ist. Die Membran schließt die vaskuläre Fehlbildung gegen das Gefäß ab, der Stent hält das Gefäß offen und sorgt dafür, dass die Membran eng an der Gefäßwand anliegt.

Ein Problem bei solchen bekannten Stentgrafts ist die sichere Verankerung der Membran am Stent. So ist beispielsweise ein Vernähen der Membran an dem Stentgerüst grundsätzlich sehr zuverlässig und haltbar jedoch auch sehr zeitaufwändig und entsprechend teuer. Alternative Entwicklungen sehen ein Fixieren der Membran an bestimmten klammerförmigen Halteelementen des Stents vor.

Eine weitere Entwicklung auf diesem Gebiet betrifft Stentgrafts in Form sogenannter Doppelstents, bei denen eine Membran zwischen zwei radial angeordneten Stents, nämlich einem äußeren und einem inneren Stent, gehalten wird. Bei der Expansion eines solchen Doppelstents nimmt die Membran an der radialen Erweiterung teil und bleibt zwischen den beiden Stents eingeklemmt.

Ein solcher Doppelstent ist beispielsweise aus der DE 197 20 115 A1 bekannt. Der bekannte Doppelstent sieht wie zuvor beschrieben eine einzelne Materialschicht bzw. Membran vor, die zwischen zwei Stents gehalten wird. Einen alternativen Doppelstent beschreibt die DE 10 2016 120 445 A1. Dort ist auf dem äußeren Stent eine zweite Membran angeordnet ist, wobei die Membranenden sowohl der ersten als auch der zweiten Membran an den Enden der Stents zusammengefasst sind, auf die Innenseite des inneren Stents umgeschlagen sind und unter Federzungen des inneren Stents festgeklemmt sind. EP 1 121 911 A2 beschreibt ein Stent-Transplantat mit einem selbstexpandierenden äußeren Stent, bei dem es sich um ein röhrenförmiges Element aus elastischem Material handelt. Das Transplantat umfasst außerdem ein röhrenförmiges, flexibles, poröses Transplantatelement, das sich entlang der Innenseite des äußeren Stents erstreckt. Das Transplantatelement hat vordere und hintere Enden, die umgefaltet und mit den vorderen und hinteren Enden des äußeren Stents verbunden werden, um Manschetten zu bilden. Darüber hinaus verfügt der Stentgraft über einen selbstexpandierenden inneren Stent, bei dem es sich ebenfalls um ein röhrenförmiges Element aus elastischem Material handelt. Der innere Stent ist so im Inneren des Transplantatelements angeordnet, dass der innere Stent, das Transplantatelement und der äußere Stent alle aneinander anliegen.

Die bekannten Doppelstents funktionieren grundsätzlich, sind jedoch in einigen Punkten verbesserungswürdig.

So ist beispielsweise die zwischen den zwei Stents angeordnete Materialschicht gemäß des zuerst beschriebenen Doppelstents während der Expansion einer Reibung zwischen innerem und äußerem Stent ausgesetzt, die zu Schäden der Materialschicht führen kann. Sollten diese Schäden sofort oder erst nach einer Weile der Beanspruchung zu Löchern oder Rissen in der Materialschicht führen, können sich Probleme mit der Dichtigkeit ergeben. Eine Abschottung beispielsweise einer vaskulären Fehlbildung wäre somit nicht mehr gegeben. Die weiterhin bekannte Variante mit zwei Membranen hat insbesondere den Nachteil, dass die jeweiligen Membranenden doppellagig innerhalb des Lumens des Doppelstents in die Federzungen eingeklemmt werden müssen. Dies ist nicht nur handwerklich anspruchsvoll, sondern führt unvermeidlich zu einer nicht unerheblichen Materialansammlung und somit zu Vorsprüngen innerhalb des Stentlumens durch die vorstehenden Klammern und durch die sich ggfs. aufwölbenden Membranenden. Solche Vorsprünge sind prädestinierte Ansatzpunkte für die Bildung von Thromben, was zu einem Verschluss des Stents führen kann.

Bei den bekannten Doppelstents ist es zudem als grundsätzlich nachteilig anzusehen, dass das innere Lumen jeweils durch einen blanken Stent ohne innere Abdeckung gebildet wird. Die offenliegenden Streben des Stents sorgen für Turbulenzen im Blutstrom und bilden eine weitere Angriffsfläche für Plaque- und Thrombenbildung, die zu einem Verschluss des Stents führen kann.

Der Erfindung liegt damit die Aufgabe zu Grunde, einen verbesserten Stentgraft bereitzustellen, der die genannten Nachteile bekannter Doppelstents nicht aufweist.

Zudem ist es Aufgabe der vorliegenden Erfindung, einen Stentgraft bereitzustellen, der einfach herzustellen ist.

Diese Aufgabe wird mit einem Mehrfachstent der eingangs genannten Art gelöst, der zumindest zwei koaxial angeordnete Stents und zumindest eine Membran umfasst, wobei ein erster Stent innenliegend und ein zweiter Stent außenliegend vorgesehen ist und eine erste Membran auf der Innenseite des ersten Stents und/oder eine zweite Membran auf der Außenseite des zweiten Stents angeordnet sind. Die jeweiligen Membranenden sind derart um die jeweiligen Enden des Stents, an dem sie angeordnet sind, umgeschlagen, dass die Membranenden zwischen zwei Stents festgeklemmt sind.

Grundsätzlich ist somit die Anordnung einer Vielzahl von Stents und daran angeordneter Membranen denkbar, als vorteilhaft haben sich jedoch im Wesentlichen die nachfolgend beschriebenen Ausführungsformen erwiesen, die zwei oder drei Stents und ein oder zwei Membranen umfassen.

Außen bzw. außenliegend ist im Sinne der Erfindung so zu verstehen, dass dieser Teil des Mehrfachstents näher an der Gefäßwand angeordnet ist, wohingegen innen bzw. innenliegend einen Teil des Mehrfachstents beschreibt, der weiter weg von der Gefäßwand und somit näher am Lumen des Mehrfachstents angeordnet ist. Näheres zu den räumlichen Angaben ergibt sich auch aus den Figuren.

In einer ersten bevorzugten Ausführungsform umfasst der erfindungsgemäße Mehrfachstent einen inneren Stent mit einem ersten und einem zweiten Ende und einen äußeren Stent mit einem ersten und einem zweiten Ende. Die Stents sind koaxial zueinander angeordnet.

In der ersten bevorzugten Ausführungsform umfasst der erfindungsgemäße Mehrfachstent weiterhin eine innere Membran und eine äußere Membran, wobei die innere Membran auf der Innenseite des inneren Stents angeordnet ist und die äußere Membran auf der Außenseite des äußeren Stents.

Die Enden der Membranen sind derart um die Enden der Stents umgeschlagen, an denen sie angeordnet sind, dass die jeweiligen Enden der Membranen zwischen den Stents eingeklemmt werden.

In einer zweiten bevorzugten Ausführungsform umfasst der erfindungsgemäße Mehrfachstent einen inneren Stent mit einem ersten und einem zweiten Ende und einen äußeren Stent mit einem ersten und einem zweiten Ende. Die Stents sind koaxial zueinander angeordnet.

In der zweiten bevorzugten Ausführungsform umfasst der erfindungsgemäße Mehrfachstent weiterhin eine innere Membran, wobei die innere Membran auf der Innenseite des inneren Stents angeordnet ist.

Die Enden der inneren Membran sind derart um die Enden des inneren Stents umgeschlagen, dass die Enden der inneren Membran zwischen den Stents eingeklemmt werden.

In einer dritten bevorzugten Ausführungsform umfasst der erfindungsgemäße Mehrfachstent einen inneren Stent mit einem ersten und einem zweiten Ende und einen äußeren Stent mit einem ersten und einem zweiten Ende. Die Stents sind koaxial zueinander angeordnet.

In der dritten bevorzugten Ausführungsform umfasst der erfindungsgemäße Mehrfachstent weiterhin eine äußere Membran, wobei die äußere Membran auf der Außenseite des äußeren Stents angeordnet ist.

Die Enden der äußeren Membran sind derart um die Enden des äußeren Stents umgeschlagen, dass die Enden der äußeren Membran zwischen den Stents eingeklemmt werden.

In einer Variante der als bevorzugt beschriebenen Ausführungsformen umfasst der Mehrfachstent jeweils einen zusätzlichen dritten bzw. mittleren Stent, der zwischen dem inneren und dem äußeren Stent angeordnet ist.

Der mittlere Stent kann zur Stabilisierung des Mehrfachstents vorgesehen sein, der mittlere Stent kann jedoch auch oder zusätzlich zur weiteren Fixierung der Membranen vorgesehen sein. Zur weiteren Fixierung der Membranen kann der mittlere Stent beispielweise eine entsprechende Oberflächenstruktur aufweisen oder aus Materialien gefertigt sein, die einem Verrutschen der Membranen entgegenwirken. Für den mittleren Stent sind entsprechend auch solche Oberflächen und Materialien denkbar, wie beispielsweise leicht angeraute Oberflächen zur Erhöhung der Reibekräfte, die für den inneren und äußeren Stent nicht vorteilhaft wären, da diese zumindest in bestimmten Ausführungsformen direkten Kontakt zur Gefäßwand bzw. zum Blutfluss haben.

In einer zusätzlichen Variante der beschriebenen Ausführungsformen ist es denkbar, dass bestimmte Stents des Mehrfachstents, also beispielsweise der innere und/oder der mittlere und/oder der äußere Stent, nicht als einheitlicher bzw. durchgehender Stentkörper vorgesehen sind, sondern sich aus einer Mehrzahl gegebenenfalls verbundener oder unverbundener Stentelemente (also einzelner Stentkörper) zusammensetzen. Diese Stentelemente können in ihrer Gesamtheit der Länge des Mehrfachstents bzw. der Länge der übrigen Stents des Mehrfachstents entsprechen, sie können aber auch insgesamt kürzer sein und somit Lücken gegenüber den übrigen Stents aufweisen oder länger sein und somit Überlappungen schaffen.

Im Sinne dieser zusätzlichen Variante sind Begriffe wie "innerer Stent" oder "äußerer Stent" besser verständlich als "innere Stentlage" oder als "äußere Stentlage" in dem Sinne, dass es sich hierbei um eine bestimmte Lage innerhalb des Mehrfachstents handelt, die aus einem einheitlichen bzw. durchgehenden Stentkörper oder aber alternativ aus einer Mehrzahl verbundener oder unverbundener Stentelemente vorgesehen sein kann.

Dabei ist vorzugsweise zumindest ein Stent des Mehrfachstents aus einem durchgehenden bzw. zusammenhängenden Stentkörper vorgesehen. Dies ist vorzugsweise der Stent, an dem die Membran vorgesehen ist.

Die nachfolgenden Ausführungen gelten für alle erfindungsgemäßen Ausführungsformen des Mehrfachstents und insbesondere für die bevorzugten Ausführungsbeispiele und beschriebenen Varianten.

Für die Membranen kann jedes dafür geeignete biologische oder künstliche Material verwandt werden. In der Regel bestehen die Membranen aus einem Kunststoff, vorzugsweise einem Kunststoffschlauch, der über den jeweiligen Stent gezogen ist. Ein geeignetes Material ist beispielsweise Polytetrafluorethylen, PTFE, insbesondere ePTFE, dass die benötigte Elastizität für die Expansion aufweist. Andere medizinisch geeignete Kunststoffe, etwa Polyester, Polyolefine, Polyurethane, Polyurethancarbonat und dergleichen, können ebenfalls eingesetzt werden. Ebenfalls denkbar sind beispielsweise aus Fäden, insbesondere Polymerfäden, gestrickte, gewebte oder genähte Membranen.

Die Länge der Membranen kann so gewählt sein, dass sich ihre Enden nach dem Umschlagen zwischen den Stents überlappen. Vorzugsweise sind die Membranen dann 5 bis 25 %, noch bevorzugter 25 bis 50 %, weiterhin bevorzugter 50 bis 75 % und insbesondere 75 bis 100 % länger als der Stent, an dem sie angeordnet sind.

Es sind jedoch auch Ausführungsformen denkbar, bei denen sich die Enden zumindest einer Membran überlappen, diese also mehr als doppelt so lang ist wie der Stent, an dem sie angeordnet ist.

Die Membranen, soweit mehrere in dem Mehrfachstent Verwendung finden, können aus unterschiedlichen Materialien vorgesehen sein und unterschiedliche Längen aufweisen.

Sofern mehrere Membranen vorgesehen sind, können diese gleichlang oder von unterschiedlicher Länge sein. Sie können symmetrisch angeordnet sein oder in einer sonstigen Konfiguration vorliegen.

Die Stents können ballonexpandierbar oder selbstexpandierbar vorgesehen sein. Entsprechend können die Stents geflochten sein oder aus einem Rohr mit geeignetem Durchmesser mithilfe eines Laserstrahls geschnitten werden. Sie verfügen über eine Maschen- bzw. Gitterstrukturstruktur.

Das erfindungsgemäße Prinzip des Mehrfachstents führt grundsätzlich zu einer relativ hohen Wandstärke des Konstrukts, was die Manövrierfähigkeit im Gefäßsystem eines Patienten einschränken kann. Dem kann dadurch begegnet werden, dass die Wandstärke der zum Schneiden der Stents verwandten Rohre oder die Durchmesser der zum Flechten der Stents verwendeten Drähte gering gewählt wird, beispielsweise im Bereich von 0,05 bis 0,50 mm, vorzugsweise 0,10 bis 0,20 mm und insbesondere etwa 0,15 mm. Auch die Stegbreite kann vermindert werden auf beispielsweise 0,05 bis 0,50 mm, vorzugsweise 0,10 bis 0,20 mm und insbesondere etwa 0,15 mm. Durch die Verwendung von zumindest zwei Stents wird auch bei geringen Wanddichten oder Drahtdurchmessern eine hohe Radialkraft erreicht.

Für die Stents kommen entsprechend die üblichen Materialien in Frage, beispielsweise medizinischer Stahl, Kobalt-Chrom-Legierungen und Nickel-Titan-Legierungen oder beliebige Kombinationen davon. Auch Kunststoffe (Polymere), beispielsweise resorbierbare Kunststoffe, darunter verschiedenste Polylactate wie sie aus dem Stand der Technik bekannt sind, können eingesetzt werden, wie auch Kombinationen aus Metallstents und Kunststoffstents. Dabei wählt der Fachmann die Anordnung und die Materialien der Stents nach den jeweiligen Aufgaben. So kann beispielsweise der innere Stent ein Metall umfassen und der äußere Stent einen Kunststoff oder auch umgekehrt. Die Stents können jedoch auch alle aus dem gleichen Material gefertigt sein.

Es versteht sich, dass die verschiedenen Stents unterschiedliche Dicken und unterschiedliche Längen aufweisen können, wobei zumindest der innere und der äußere Stent des erfindungsgemäßen Mehrfachstents vorzugsweise symmetrisch zueinander angeordnet sind.

Denkbar sind jedoch auch Ausführungsformen, bei denen die Stents gegeneinander versetzt angeordnet sind oder unterschiedliche Längen aufweisen, soweit hierdurch die erforderliche Klemmwirkung noch erreicht wird.

Ebenso können für die Stents unterschiedliche Designs verwandt werden. So ist es beispielsweise bevorzugt, weiter außen liegende Stents bzw. den äußeren Stent mit kleineren Maschen zu versehen als weiter innen liegende Stents bzw. den inneren Stent. Hierdurch wird bei der Expansion eine Pressspannung erzeugt, die sich vorteilhaft auf die Radialkraft und den Zusammenhalt des Konstrukts auswirkt. Erzielt wird eine hohe Festigkeit und Dauerhaftigkeit des Konstrukts.

Der nominale Durchmesser der weiter innen liegenden Stents bzw. des inneren Stents ist mindestens genauso groß wie der der weiter außen liegenden Stents, wobei der nominale Durchmesser der weiter innen liegenden Stents vorzugsweise sogar etwas größer gewählt werden sollte als der der weiter außen liegenden Stents, um so eine größere Klemmwirkung zu erzielen.

Das Festklemmen der Membranenden zwischen den Stents führt zu einer zuverlässigen Verankerung der Membran und ist einfach in der Herstellung, da keine kleinteiligen Klemmelemente hergestellt und bedient oder aufwändige Näharbeiten durchgeführt werden müssen.

In einer Weiterbildung des erfindungsgemäßen Mehrfachstents ist dieser insbesondere zum Einsatz in Multilumenimplantaten geeignet. Unter einem Multilumenimplantat ist ein solches Implantat zu verstehen, dass zur Implantation im Gefäßsystem vorgesehen ist und an die Gefäßmorphologie des Patienten angepasste Verzweigungen umfasst. In der Regel handelt es sich hierbei um mehrlumige Stentgrafts.

Multilumenimplantate werden häufig als Kits angeboten, da sich die individuelle Gefäßmorphologie von Patient zu Patient stark unterscheiden kann und Einzelanfertigungen teuer und zeitaufwändig sind. Entsprechend werden während oder im Vorfeld der Intervention die einzelnen Äste des Multilumenimplantats auf die jeweiligen Durchmesser der zu behandelnden Gefäße abgestimmt ausgewählt. Kritisch ist hierbei die sichere und einfach herzustellende Verbindung der einzelnen Komponenten.

Die Weiterbildung sieht entsprechend zumindest an einem Ende bzw. an einem Randbereich des Mehrfachstents die Anordnung von Haltelementen vor, mit denen der Mehrfachstent in einem Multilumenimplantat festlegbar ist. Die Halteelemente können beispielsweise in Form von hakenförmigen und radial nach außen gerichteten Elementen vorgesehen sein. Diese hakenförmigen und radial nach außen gerichteten Elemente können vorzugsweise Teil des blanken, also nicht mit einer Membran überzogenen Stents sein.

Als Halteelemente können auch bestimmte Umformungen der Stentenden bzw. der Randbereiche der Stents dienen. So ist es beispielsweise denkbar, dass sich zumindest ein Endbereich mindestens eines Stents, der Teil des Mehrfachstents ist, nach außen hin erweitert. Hierzu können die endständigen Streben des Stents beispielsweise besonders ausgeformt sein, insbesondere können diese länger sein als die übrigen Streben, um eine solche Umformung zu begünstigen.

Es ist auch denkbar, dass insbesondere die hakenförmigen Halteelemente in einer Doppelfunktion vorgesehen sind, indem sie an dem gecoverten Stent vorgesehen sind und neben ihrer Haltefunktion im Multilumenimplantat eine zusätzliche Fixierung der Membran am Umschlagspunkt ermöglichen. Die Halteelemente sind in jedem Fall so anzuordnen, dass sie ausreichend weit von dem Mehrfachstent abstehen, um eine ausreichende Verankerung in einem Multilumenimplantat zu ermöglichen.

Hierzu kann beispielsweise das mit Haltelementen versehene Ende des Stents etwas länger sein und so bereits aus dem Mehrfachstent herausragen, was insbesondere dann sinnvoll ist, wenn einer der inneren Stents mit Haltelementen ausgestattet wird.

Sofern der äußere Stent mit hakenförmigen Halteelementen ausgestattet wird, so ist es insbesondere in Hinblick auf die zweite beschriebene Ausführungsform bevorzugt, den ungecoverten äußeren Stent insgesamt kürzer vorzusehen als den inneren gecoverten Stent, sodass der äußere Stent gegenüber dem Gefäßlumen vollständig durch den inneren Stent bzw. durch die auf diesem Stent angeordnete Membran überdeckt wird.

Neben den erwähnten Haken sind weitere Ausgestaltungen der Halteelemente denkbar, beispielsweise auch solche, die speziell auf entsprechende Ansatzstellen im Multilumenimplantat abgestimmt sind, ähnlich einem Schlüssel-Schloss-Prinzip oder einem Haken-Öse-Prinzip.

Der Verbund eines inneren und eines äußeren Stents mit einer äußeren und/oder einer inneren Membran führt zu einem überraschend stabilen Implantat, das dennoch über eine hohe Flexibilität verfügt. Die Membranen und die zumindest doppelte Stentlage tragen zur Stabilisierung des Mehrfachstents bei und erlauben es, die Stentwände vergleichsweise dünn zu halten. Dennoch verfügt der Mehrfachstent über eine gute Radialkraft.

Die Erfindung wird durch die beiliegenden Abbildungen näher erläutert, die bevorzugte Ausführungsformen der Erfindung zeigen. Es versteht sich, dass in den Abbildungen gezeigten Merkmale jeweils für sich Teil der Erfindung sind und nicht nur im Zusammenhang mit anderen Merkmalen der Abbildung zu verstehen sind.

Es zeigen:
- Figur 1: den schematisch den Aufbau einer ersten Ausführungsform des erfindungsgemäßen Mehrfachstents;
- Figur 2: Detail B aus Figur 1 mit der Anordnung der Stents und Membranen gemäß der ersten Ausführungsform des erfindungsgemäßen Mehrfachstents;
- Figur 3: den schematisch den Aufbau einer zweiten Ausführungsform des erfindungsgemäßen Mehrfachstents;
- Figur 4: Detail B aus Figur 3 mit der Anordnung der Stents und der Membran gemäß der zweiten Ausführungsform des erfindungsgemäßen Mehrfachstents;
- Figur 5: den schematisch den Aufbau einer dritten Ausführungsform des erfindungsgemäßen Mehrfachstents;
- Figur 6: Detail B aus Figur 5 mit der Anordnung der Stents und der Membran gemäß der dritten Ausführungsform des erfindungsgemäßen Mehrfachstents;
- Figur 7: den Einsatz des erfindungsgemäßen Mehrfachstents in einem Multilumenimplantat;
- Figur 8: Detail einer Weiterbildung des erfindungsgemäßen Mehrfachstents mit Haltelementen zum Einsatz in einem Multilumenimplantat;

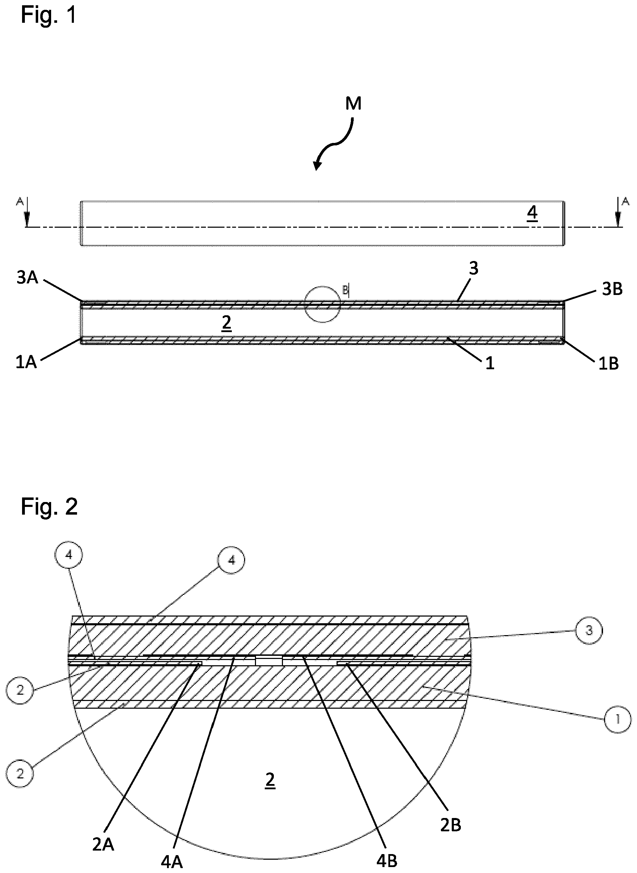

Figur 1 zeigt eine erste Ausführungsform des erfindungsgemäßen Mehrfachstents M mit einem inneren Stent 1 mit einem ersten 1A und einem zweiten Ende 1B und einem äußeren Stent 3 mit einem ersten 3A und einem zweiten Ende 3B. Die Stents 1, 3 sind koaxial zueinander angeordnet. Der Mehrfachstent M ist im nicht expandierten Zustand dargestellt. Auf der Innenseite des inneren Stents 1 ist eine innere Membran 2 angeordnet und auf der Außenseite des äußeren Stents 3 ist eine äußere Membran 4 angeordnet.

Die Membranen 2, 4 sind um die Enden 1A, 1B, 3A, 3B der Stents 1, 3 umgeschlagen, sodass sie mit ihren Enden jeweils zwischen den Stents 1, 3 eingeklemmt werden.

Die Stents 1, 3 können ballonexpandierbar oder selbstexpandierbar vorgesehen sein, wobei alle bekannten Materialien einsetzbar sind. Die Stents 1, 3 können aus unterschiedlichen Materialien vorgesehen sein. Der nominale Durchmesser des inneren Stents 1 ist mindestens genauso groß wie der des äußeren Stents 3, wobei der nominale Durchmesser des inneren Stents 1 vorzugsweise sogar etwas größer gewählt werden sollte als der des äußeren Stents 3, um eine größere Klemmwirkung zu erzielen.

Die Membranen 2, 4 können aus den unterschiedlichsten bekannten Materialien gefertigt sein, wobei schlauchförmige Membranen aus ePTFE bevorzugt sind. Die Membranen 2, 4 können auch aus unterschiedlichen Materialien vorgesehen sein.

Figur 2 zeigt eine Detailansicht B aus Figur 1 zur Anordnung der Stents 1, 3 und der Membranen 2, 4 zueinander. Die innere Membran 2 und die äußere Membran 4 werden mit ihren beiden Enden 2A, 2B, 4A, 4B jeweils um das Ende 1A, 1B, 3A, 3B des Stents 1, 3, dem sie innen bzw. außen anliegen, in den Bereich zwischen den Stents 1, 3 umgeschlagen. Die Enden 2A, 2B, 4A, 4B bzw. genauer die Endbereiche der Membranen 2, 4 werden somit zwischen den beiden Stents 1, 3 eingeklemmt. Die Membranen 2, 4 können, wie dargestellt, eine unterschiedliche Länge aufweisen, sie können aber auch gleichlang vorgesehen sein. Die Länge der Membranen 2, 4 kann, wie dargestellt, so gewählt sein, dass sich die jeweiligen Enden 2A, 2B bzw. 4A, 4B zwischen den Stents 1, 3 nicht berühren bzw. überlappen, die Länge der Membranen 2, 4 kann aber auch so gewählt sein, dass sich die jeweiligen Enden 2A, 2B bzw. 4A, 4B zwischen den Stents 1, 3 berühren oder überlappen.

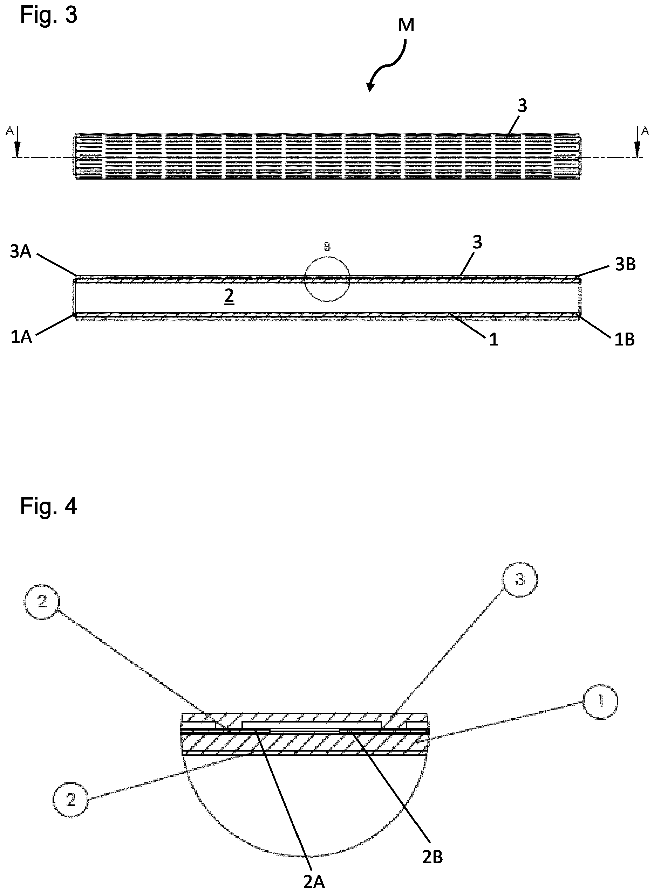

Figur 3 zeigt eine zweite Ausführungsform des erfindungsgemäßen Mehrfachstents M mit einem ersten inneren Stent 1 mit einem ersten 1A und einem zweiten Ende 1B und einem zweiten äußeren Stent 3 mit einem ersten 3A und einem zweiten Ende 3B. Die Stents 1, 3 sind koaxial zueinander angeordnet. Der Mehrfachstent M ist im nicht expandierten Zustand dargestellt. Bei dieser Ausführungsform ist nur eine Membran 2 auf der Innenseite des inneren Stents 1 angeordnet.

Die Membran 2 ist um die Enden 1A, 1B des inneren Stents 1 umgeschlagen, sodass sie mit ihren Enden zwischen den beiden Stents 1, 3 eingeklemmt wird.

Die Stents 1, 3 können ballonexpandierbar oder selbstexpandierbar vorgesehen sein, wobei alle bekannten Materialien einsetzbar sind. Die Stents 1, 3 können aus unterschiedlichen Materialien vorgesehen sein. Der nominale Durchmesser des inneren Stents 1 ist mindestens genauso groß wie der des äußeren Stents 3, wobei der nominale Durchmesser des inneren Stents 1 vorzugsweise sogar etwas größer gewählt werden sollte als der des äußeren Stents 3, um eine größere Klemmwirkung zu erzielen.

Die Membran 2 kann aus den unterschiedlichsten bekannten Materialien gefertigt sein, wobei schlauchförmige Membranen aus ePTFE bevorzugt sind.

Figur 4 zeigt eine Detailansicht B aus Figur 3 zur Anordnung der Stents 1, 3 und der Membran 2 zueinander. Die innere Membran 2 wird mit ihren beiden Enden 2A, 2B jeweils um das Ende 1A, 1B des inneren Stents 1 in den Bereich zwischen den Stents 1, 3 umgeschlagen. Die Enden 2A, 2B der Membran 2 werden somit zwischen den beiden Stents 1, 3 eingeklemmt. Die Länge der Membran 2 kann, wie dargestellt, so gewählt sein, dass sich ihre Enden 2A, 2B zwischen den Stents 1, 3 nicht berühren bzw. überlappen, die Länge der Membran 2 kann aber auch so gewählt sein, dass sich ihre Enden 2A, 2B zwischen den Stents 1, 3 berühren oder überlappen.

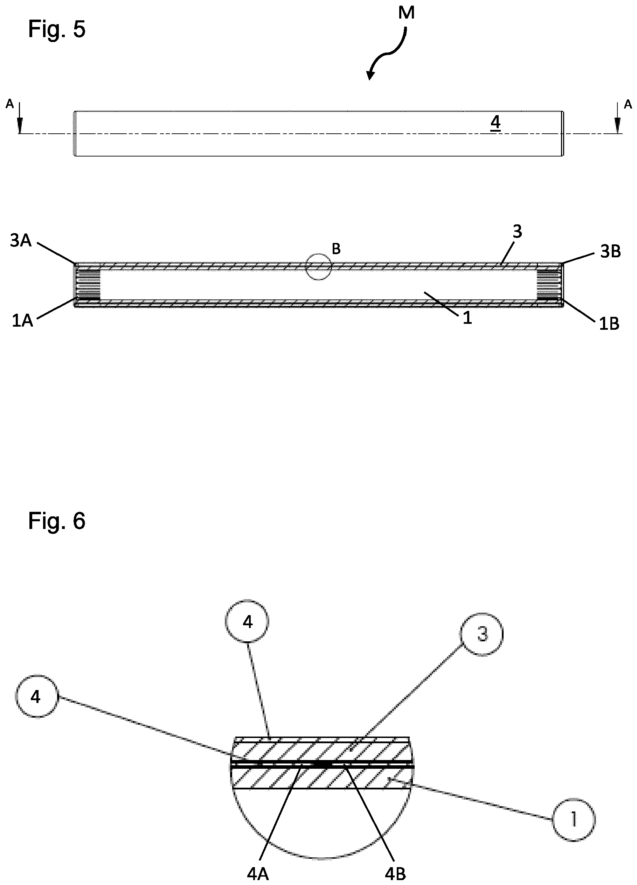

Figur 5 zeigt eine dritte Ausführungsform des erfindungsgemäßen Mehrfachstents M mit einem ersten inneren Stent 1 mit einem ersten 1A und einem zweiten Ende 1B und einem zweiten äußeren Stent 3 mit einem ersten 3A und einem zweiten Ende 3B. Die Stents 1, 3 sind koaxial zueinander angeordnet. Der Mehrfachstent M ist im nicht expandierten Zustand dargestellt. Bei dieser Ausführungsform ist nur eine Membran 4 auf der Außenseite des äußeren Stents 3 angeordnet.

Die Membran 4 ist um die Enden 3A, 3B des äußeren Stents 3 umgeschlagen, sodass sie mit ihren Enden zwischen den beiden Stents 1, 3 eingeklemmt wird.

Die Stents 1, 3 können ballonexpandierbar oder selbstexpandierbar vorgesehen sein, wobei alle bekannten Materialien einsetzbar sind. Die Stents 1, 3 können aus unterschiedlichen Materialien vorgesehen sein.

Der nominale Durchmesser des inneren Stents 1 ist mindestens genauso groß wie der des äußeren Stents 3, wobei der nominale Durchmesser des inneren Stents 1 vorzugsweise sogar etwas größer gewählt werden sollte als der des äußeren Stents 3, um eine größere Klemmwirkung zu erzielen.

Die Membran 4 kann aus den unterschiedlichsten bekannten Materialien gefertigt sein, wobei schlauchförmige Membranen aus ePTFE bevorzugt sind.

Figur 6 zeigt eine Detailansicht B aus Figur 5 zur Anordnung der Stents 1, 3 und der Membran 4 zueinander. Die äußere Membran 4 wird mit ihren beiden Enden 4A, 4B jeweils um das Ende 3A, 3B des äußeren Stents 3 in den Bereich zwischen den Stents 1, 3 umgeschlagen. Die Enden 4A, 4B der Membran 4 werden somit zwischen den beiden Stents 1, 3 eingeklemmt.

Die Länge der Membran 4 kann, wie dargestellt, so gewählt sein, dass sich ihre Enden 4A, 4B zwischen den Stents 1, 3 nicht berühren bzw. überlappen, die Länge der Membran 4 kann aber auch so gewählt sein, dass sich ihre Enden 4A, 4B zwischen den Stents 1, 3 berühren oder überlappen.

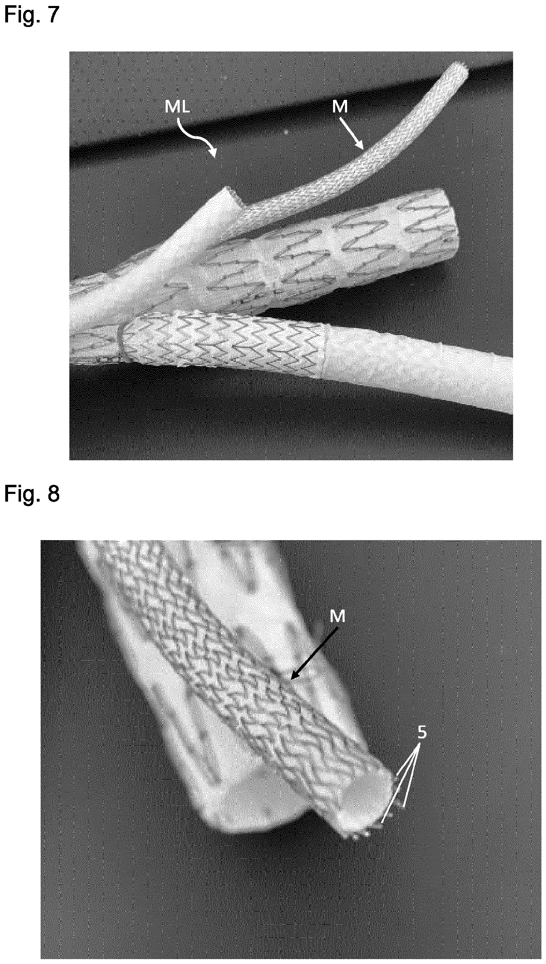

Figur 7 zeigt den Einsatz einer Weiterbildung des erfindungsgemäßen Mehrfachstents M in einem Multilumenimplantat ML, also in einem verzweigten Stentgraft.

Figur 8 zeigt im Detail den Endbereich einer Weiterbildung des erfindungsgemäßen Mehrfachstents M zum Einsatz in einem Multilumenimplantat ML gemäß Figur 7. Die Weiterbildung sieht zumindest an einem Ende des Mehrfachstents M die Anordnung von Haltelementen 5 vor, mit denen der Mehrfachstent M in einem Multilumenimplantat ML festlegbar ist. Multilumenimplantate ML werden häufig als Kits angeboten, wobei die einzelnen Äste des Multilumenimplantats ML abgestimmt auf die jeweiligen Durchmesser der zu behandelnden Gefäße ausgewählt werden. Kritisch ist die Verbindung der einzelnen Komponenten. Hierfür sieht die Weiterbildung vorzugsweise Halteelemente 5 in Form von hakenförmigen und radial nach außen gerichteten Elementen 5 vor. Diese können vorzugsweise Teil des nicht gecoverten Stents sein, da die Enden des gecoverten Stents von der umgeschlagenen Membran abgedeckt sind. Ein Durchstoßen der Membran durch die Halteelemente würde zu einer unnötigen Materialbeschädigung führen. Es ist jedoch auch denkbar, dass die Halteelemente 5 in einer Doppelfunktion vorgesehen sind, indem sie an dem gecoverten Stent vorgesehen sind und neben ihrer Haltefunktion im Multilumenimplantat ML eine zusätzliche Fixierung der Membran am Umschlagspunkt ermöglichen. Die Halteelemente 5 sind in jedem Fall so anzuordnen, dass sie ausreichend weit hervorstehen, um eine ausreichende Verankerung in einem Multilumenimplantat ML zu ermöglichen.

### Bezugszeichenliste

- 1: innerer (erster) Stent (1A, 1B: Enden des inneren Stents)
- 2: innere (erste) Membran (2A, 2B: Enden der inneren Membran)
- 3: äußerer (zweiter) Stent (3A, 3B: Enden des äußeren Stents)
- 4: äußere (zweite) Membran (4A, 4B: Enden der äußeren Membran)
- 5: Halteelemente
- M: Mehrfachstent
- ML: Multilumenimplantat

## Patentansprüche

1. Mehrfachstent mit zumindest zwei koaxial angeordneten Stents (1, 3) und zumindest einer Membran (2, 4), wobei ein erster Stent (1) innenliegend und ein zweiter Stent (3) außenliegend vorgesehen ist, **dadurch gekennzeichnet, dass** eine erste Membran (2) auf der Innenseite des ersten Stents (1) und/oder eine zweite Membran (4) auf der Außenseite des zweiten Stents (3) angeordnet sind, wobei die jeweiligen Membranenden (2A, 2B; 4A, 4B) derart um die jeweiligen Enden (1A, 1B; 3A, 3B) des Stents (1, 3), an dem sie angeordnet sind, umgeschlagen sind, dass die Membranenden (2A, 2B; 4A, 4B) zwischen den ersten und den zweiten Stent (1, 3) geführt sind.

2. Mehrfachstent nach Anspruch 1, **dadurch gekennzeichnet, dass** die weiter innen angeordneten Stents (1) einen mindestens gleichgroßen nominalen Durchmesser aufweisen wie die weiter außen angeordneten Stents (3), wobei die weiter innen angeordneten Stents (1) vorzugsweise einen größeren nominalen Durchmesser aufweisen als die weiter außen angeordneten Stents (3).

3. Mehrfachstent nach einem der vorherigen Ansprüche, **dadurch gekennzeichnet, dass** die mindestens eine Membran (2, 4) schlauchförmig vorgesehen ist.

4. Mehrfachstent nach einem der vorherigen Ansprüche, **dadurch gekennzeichnet, dass** die mindestens eine Membran (2, 4) 5 bis 25 % länger, vorzugsweise 25 bis 50 % länger, weiterhin bevorzugt 50 bis 75 % und insbesondere 75% bis 100% länger ist als der Stent (1, 3), an dem sie angeordnet ist.

5. Mehrfachstent nach einem der vorherigen Ansprüche, **dadurch gekennzeichnet, dass** die mindestens eine Membran (2, 4) mehr als doppelt so lang ist wie der Stent (1, 3), an dem sie angeordnet ist, sodass sich die umgeklappten Membranenden (2A, 2B; 4A, 4B) zwischen den Stents (1, 3) jeweils überlappen.

6. Mehrfachstent nach einem der vorherigen Ansprüche, **wobei** die Membranen (2, 4) aus einer Folie gefertigt oder aus einem Faden gestrickt, gewebt oder genäht vorgesehen sein können.

7. Mehrfachstent nach einem der Ansprüche 1 bis 6 mit zwei koaxial angeordneten Stents (1, 3) und zwei Membranen (2, 4), wobei der erste Stent (1) innenliegend und der zweite Stent (3) außenliegend vorgesehen sind, **dadurch gekennzeichnet, dass** die erste Membran (2) auf der Innenseite des ersten Stents (1) angeordnet ist und die Membranenden (2A, 2B) der ersten Membran (2) auf die Außenseite des ersten Stents (1) umgeschlagen sind und die zweite Membran (4) auf der Außenseite des zweiten Stents (3) angeordnet ist und die Membranenden (4A, 4B) der zweiten Membran (4) auf die Innenseite des zweiten Stents (3) umgeschlagen sind, sodass die Membranenden (2A, 2B; 4A, 4B) zwischen den Stents (1, 3) festgeklemmt sind.

8. Mehrfachstent nach einem der Ansprüche 1 bis 6 mit zwei koaxial angeordneten Stents (1, 3) und einer Membran (2), wobei der erste Stent (1) innenliegend und der zweite Stent (3) außenliegend vorgesehen sind, **dadurch gekennzeichnet, dass** die Membran (2) auf der Innenseite des ersten Stents (1) angeordnet ist und die Membranenden (2A, 2B) auf die Außenseite des ersten Stents (1) umgeschlagen sind, sodass die Membranenden (2A, 2B) zwischen den Stents (1, 3) festgeklemmt sind.

9. Mehrfachstent nach einem der Ansprüche 1 bis 6 mit zwei koaxial angeordneten Stents (1, 3) und einer Membran (4), wobei der erste Stent (1) innenliegend und der zweite Stent (3) außenliegend vorgesehen sind, **dadurch gekennzeichnet, dass** die Membran (4) auf der Außenseite des zweiten Stents (3) angeordnet ist und die Membranenden (4A, 4B) auf die Innenseite des zweiten Stents (3) umgeschlagen sind, sodass die Membranenden (4A, 4B) zwischen den Stents (1, 3) festgeklemmt sind.

10. Mehrfachstent nach einem der Ansprüche 1 bis 6 mit drei koaxial angeordneten Stents und zwei Membranen, wobei der erste Stent innenliegend, der zweite Stent außenliegend und der dritte Stent zwischen dem ersten und dem zweiten Stent liegend vorgesehen sind, **dadurch gekennzeichnet, dass** die erste Membran auf der Innenseite des ersten Stents angeordnet ist und die Membranenden der ersten Membran auf die Außenseite des ersten Stents umgeschlagen sind und zwischen dem ersten und dem dritten Stent eingeklemmt sind und die zweite Membran auf der Außenseite des zweiten Stents angeordnet ist und die Membranenden der zweiten Membran auf die Innenseite des zweiten Stents umgeschlagen sind und zwischen dem zweiten und dem dritten Stent eingeklemmt sind.

11. Mehrfachstent nach einem der vorstehenden Ansprüche, wobei zumindest an einem Ende eines Stents des Mehrfachstents (M) Verankerungselemente (5) vorgesehen sind.

12. Mehrfachstent nach Anspruch 11, wobei die Verankerungselemente (5) als Erweiterung zumindest eines Endbereichs mindestens eines Stents des Mehrfachstents (M) vorgesehen sind.

13. Mehrfachstent nach Anspruch 11, wobei die Verankerungselemente (5) hakenförmig und radial nach außen gerichtet vorgesehen sind.

14. Mehrfachstent nach einem der vorstehenden Ansprüche, **dadurch gekennzeichnet, dass** zumindest ein Stent des Mehrfachstents (M) eine Vielzahl einzelner Stentelemente umfasst.

## Claims

1. Multiple stent comprising at least two coaxially arranged stents (1, 3) and at least one membrane (2, 4), wherein a first stent (1) being arranged on the inside and a second stent (3) being arranged on the outside, **characterized in that** a first membrane (2) is arranged on the inside of the first stent (1) and/or a second membrane (4) is arranged on the outside of the second stent (3), wherein the respective membrane ends (2A, 2B; 4A, 4B) are folded around the respective ends (1A, 1B; 3A, 3B) of the stent (1, 3) on which they are arranged, in such a way that the membrane ends (2A, 2B; 4A, 4B) are guided between the first and the second stent (1, 3).

2. Multiple stent according to claim 1, **characterized in that** the more inwardly arranged stents (1) have a nominal diameter of at least the same size as the more outwardly arranged stents (3), wherein the more inwardly arranged stents (1) preferably have a larger nominal diameter than the more outwardly arranged stents (3).

3. Multiple stent according to any one of the preceding claims, **characterized in that** the at least one membrane (2, 4) is provided in tubular form.

4. Multiple stent according to any one of the preceding claims, **characterized in that** the at least one membrane (2, 4) is 5 to 25% longer, preferably 25 to 50% longer, further preferably 50 to 75% and in particular 75% to 100% longer than the stent (1, 3) on which it is arranged.

5. Multiple stent according to any one of the preceding claims, **characterized in that** the at least one membrane (2, 4) is more than twice as long as the stent (1, 3) on which it is arranged, so that the folded-over membrane ends (2A, 2B; 4A, 4B) overlap between the stents (1, 3), respectively.

6. Multiple stent according to any one of the preceding claims, **wherein** the membranes (2, 4) may be made of a film or provided knitted, woven or sewn from a thread.

7. Multiple stent according to any one of claims 1 to 6 comprising two coaxially arranged stents (1, 3) and two membranes (2, 4), with the first stent (1) being provided on the inside and the second stent (3) being provided on the outside, **characterized in that** the first membrane (2) is arranged on the inside of the first stent (1) and the membrane ends (2A, 2B) of the first membrane (2) are folded over to the outside of the first stent (1) and the second membrane (4) is arranged on the outside of the second stent (3) and the membrane ends (4A, 4B) of the second membrane (4) are folded over to the inside of the second stent (3) so that the membrane ends (2A, 2B; 4A, 4B) are clamped in place between the stents (1, 3).

8. Multiple stent according to any one of claims 1 to 6, comprising two coaxially arranged stents (1, 3) and one membrane (2), wherein the first stent (1) is provided on the inside and the second stent (3) is provided on the outside, **characterized in that** the membrane (2) is arranged on the inside of the first stent (1) and the membrane ends (2A, 2B) are folded over onto the outside of the first stent (1) so that the membrane ends (2A, 2B) are clamped in place between the stents (1, 3).

9. Multiple stent according to any one of claims 1 to 6 with two coaxially arranged stents (1, 3) and one membrane (4), wherein the first stent (1) is provided on the inside and the second stent (3) is provided on the outside, **characterized in that** the membrane (4) is arranged on the outside of the second stent (3) and the membrane ends (4A, 4B) are folded over onto the inside of the second stent (3) so that the membrane ends (4A, 4B) are clamped in place between the stents (1, 3).

10. Multiple stent according to any one of claims 1 to 6, comprising three coaxially arranged stents and two membranes, wherein the first stent being arranged on the inside, the second stent being provided on the outside, and the third stent being provided between the first stent and the second stent, **characterized in that** the first membrane is arranged on the inside of the first stent and the membrane ends of the first membrane are folded over to the outside of the first stent and are clamped in place between the first and the third stent and the second membrane is arranged on the outside of the second stent and the membrane ends of the second membrane are folded over to the inside of the second stent and are clamped in place between the second and the third stent.

11. Multiple stent according to any of the preceding claims, wherein anchoring elements (5) are provided at least at one end of a stent of the multiple stent (M).

12. Multiple stent according to claim 11, wherein the anchoring elements (5) are provided as an extension of at least one end portion of at least one stent of the multiple stent (M).

13. Multiple stent according to claim 11, wherein the anchoring elements (5) are provided to be of hook-shaped configuration and directed radially outward.

14. Multiple stent according to any one of the preceding claims, **characterized in that** at least one stent of the multiple stent (M) comprises a plurality of individual stent elements.

## Revendications

1. Endoprothèse multiple comportant au moins deux endoprothèses (1, 3) disposées de manière coaxiale et au moins une membrane (2, 4), une première endoprothèse (1) étant située à l'intérieur et une deuxième endoprothèse (3) à l'extérieur, **caractérisée en ce qu'**une première membrane (2) est disposée sur le côté intérieur de la première endoprothèse (1) et/ou une deuxième membrane (4) est disposée sur le côté extérieur de la deuxième endoprothèse (3), les extrémités de membrane (2A, 2B ; 4A, 4B) respectives étant repliées autour des extrémités (1A, 1B ; 3A, 3B) respectives de l'endoprothèse (1, 3) sur laquelle elles sont disposées, de telle manière que les extrémités de membrane (2A, 2B ; 4A, 4B) sont guidées entre la première et la deuxième endoprothèse (1, 3).

2. Endoprothèse multiple selon la revendication 1, **caractérisée en ce que** les endoprothèses (1) disposées davantage à l'intérieur présentent un diamètre nominal au moins égal à celui des endoprothèses (3) disposées davantage à l'extérieur, les endoprothèses (1) disposées davantage à l'intérieur présentant de préférence un diamètre nominal supérieur à celui des endoprothèses (3) disposées davantage à l'extérieur.

3. Endoprothèse multiple selon l'une des revendications précédentes, **caractérisée en ce que** l'au moins une membrane (2, 4) présente une forme de tuyau.

4. Endoprothèse multiple selon l'une des revendications précédentes, **caractérisée en ce que** l'au moins une membrane (2, 4) est 5 à 25 % plus longue, de préférence 25 à 50 % plus longue, de préférence encore 50 à 75 % et en particulier 75 % à 100 % plus longue que l'endoprothèse (1, 3) sur laquelle elle est disposée.

5. Endoprothèse multiple selon l'une des revendications précédentes, **caractérisée en ce que** l'au moins une membrane (2, 4) est plus de deux fois plus longue que l'endoprothèse (1, 3) sur laquelle elle est disposée, de telle sorte que les extrémités de membrane (2A, 2B ; 4A, 4B) repliées se chevauchent respectivement entre les endoprothèses (1, 3).

6. Endoprothèse multiple selon l'une des revendications précédentes, dans laquelle il est possible de prévoir que les membranes (2, 4) soient fabriquées à partir d'une feuille ou tricotées, tissées ou cousues à partir d'un fil.

7. Endoprothèse multiple selon l'une des revendications 1 à 6, comportant deux endoprothèses (1, 3) disposées de manière coaxiale et deux membranes (2, 4), la première endoprothèse (1) étant située à l'intérieur et la deuxième endoprothèse (3) à l'extérieur, **caractérisée en ce que** la première membrane (2) est disposée sur le côté intérieur de la première endoprothèse (1) et les extrémités de membrane (2A, 2B) de la première membrane (2) sont repliées sur le côté extérieur de la première endoprothèse (1) et la deuxième membrane (4) est disposée sur le côté extérieur de la deuxième endoprothèse (3) et les extrémités de membrane (4A, 4B) de la deuxième membrane (4) sont repliées sur le côté intérieur de la deuxième endoprothèse (3), de telle sorte que les extrémités de membrane (2A, 2B ; 4A, 4B) sont serrées entre les endoprothèses (1, 3).

8. Endoprothèse multiple selon l'une des revendications 1 à 6, comportant deux endoprothèses (1, 3) disposées de manière coaxiale et une membrane (2), la première endoprothèse (1) étant située à l'intérieur et la deuxième endoprothèse (3) à l'extérieur, **caractérisée en ce que** la membrane (2) est disposée sur le côté intérieur de la première endoprothèse (1) et les extrémités de membrane (2A, 2B) sont repliées sur le côté extérieur de la première endoprothèse (1), de telle sorte que les extrémités de membrane (2A, 2B) sont serrées entre les endoprothèses (1, 3).

9. Endoprothèse multiple selon l'une des revendications 1 à 6, comportant deux endoprothèses (1, 3) disposées de manière coaxiale et une membrane (4), la première endoprothèse (1) étant située à l'intérieur et la deuxième endoprothèse (3) à l'extérieur, **caractérisée en ce que** la membrane (4) est disposée sur le côté extérieur de la deuxième endoprothèse (3) et les extrémités de membrane (4A, 4B) sont repliées sur le côté intérieur de la deuxième endoprothèse (3), de telle sorte que les extrémités de membrane (4A, 4B) sont serrées entre les endoprothèses (1, 3).

10. Endoprothèse multiple selon l'une des revendications 1 à 6, comportant trois endoprothèses disposées de manière coaxiale et deux membranes, la première endoprothèse étant située à l'intérieur, la deuxième endoprothèse à l'extérieur et la troisième endoprothèse entre la première et la deuxième endoprothèse, **caractérisée en ce que** la première membrane est disposée sur le côté intérieur de la première endoprothèse et les extrémités de membrane de la première membrane sont repliées sur le côté extérieur de la première endoprothèse et sont coincées entre la première et la troisième endoprothèse et la deuxième membrane est disposée sur le côté extérieur de la deuxième endoprothèse et les extrémités de membrane de la deuxième membrane sont repliées sur le côté intérieur de la deuxième endoprothèse et sont coincées entre la deuxième et la troisième endoprothèse.

11. Endoprothèse multiple selon l'une des revendications précédentes, dans laquelle des éléments d'ancrage (5) sont situés au moins sur une extrémité d'une endoprothèse de l'endoprothèse multiple (M).

12. Endoprothèse multiple selon la revendication 11, dans laquelle les éléments d'ancrage (5) se présentent sous la forme d'une extension d'au moins une zone d'extrémité d'au moins une endoprothèse de l'endoprothèse multiple (M).

13. Endoprothèse multiple selon la revendication 11, dans laquelle les éléments d'ancrage (5) se présentent sous la forme de crochets et sont orientés radialement vers l'extérieur.

14. Endoprothèse multiple selon l'une des revendications précédentes, **caractérisée en ce qu'**au moins une endoprothèse de l'endoprothèse multiple (M) comprend une pluralité d'éléments d'endoprothèse individuels.
